# EUROPEAN PATENT APPLICATION

(11) **EP 4 487 768 A1**
(43) Date of publication of application: **08.01.2025**
(21) Application number: 23183340.1
(22) Date of filing: 04.07.2023
(51) Int. Cl.: A61B 5/11, A61B 5/37, A61B 5/374, G16H 50/20, G16H 20/40

(54) **THERAPY FOR THE TREATMENT OF DISORDERS IN THE NERVOUS SYSTEM BY MEANS OF TREMOR DETECTION THROUGH DEEP LEARNING**

(71) Applicant: BIOTRONIK SE & Co. KG, 12359 Berlin (DE)
(72) Inventor: Schulze-Luckow, Sebastian, 10715 Berlin (DE); Kibler, Andrew B., Lake Oswego, 97035 (US)
(74) Representative: Biotronik Corporate Services SE

(57) **Abstract**

The present invention relates to a method, comprising the steps of: obtaining, by a computing system (1), one or more electroencephalograms (EEG) of a spontaneous electrical activity of the brain (B) of a patient (P), preprocessing, by the computing system (1), the one or more electroencephalograms; and applying, by the computing system (1), a deep learning model to the preprocessed one or more electroencephalograms to generate tremor data indicating whether the one or more electroencephalograms represent one or more occurrences of one or more tremors.

## Description

The present invention relates to computing systems and methods for detecting tremors in patients.

Typical symptoms of diseases like Parkinson's are tremors. A tremor is an involuntary, often rhythmic, muscle contraction and relaxation involving back and forth movements of one or more body parts. It is one of the most common involuntary movements, and frequently occurs in the hands of a patient, but it can also affect other body parts such as arms, eyes, face, head, vocal folds, trunk, and legs.

Known solutions for diagnosing/treating tremors require visiting a physician for clinical presentation, magnetic resonance therapy of the brain after medical diagnosis, and measurement of muscle activity by means of electrodes in the clinic.

However, all of the above measures typically involve visiting a physician. The typical population of patients is regularly older than 65 years, the repeated visit of the doctor is necessary for the diagnosis of this population. Thus, particularly, only a temporary measurement (time window) is provided, not a permanent monitoring, so that an insufficient diagnosis is a possible outcome. Furthermore, the diagnosis depends on the experience of the physician, wherein such diagnosis usually confirms that a tremor is either present or not, and may describe the quality of the tremor as severe or mild - but will regularly not be given as objectively measured value. Therefore, existing solutions are often partially unnecessary, expensive, time consuming, i.e. they tie up personnel, not comparable to the advance of the state of knowledge, and possibly prone to error as the physician evaluates.

Therefore, based on the above, the problem to be solved by the present invention is to provide methods and computing systems that allow to improve recognition, particularly detecting and/or predicting, of involuntary body movements such as tremors.

This problem is solved by a method having the features of claim 1 as well as by a computing system having the features of claim 11, a computer-readable storage medium having the features of claim 13, a method having the features of claim 15, a computing system having the features of claim 20. Preferred embodiments of these aspects of the present invention are stated in the corresponding dependent claims and are described below.

According to claim 1 a method is disclosed, the method comprising the steps of:
- obtaining, by a computing system, one or more electroencephalograms (EEG) that each represent a waveform of a spontaneous electrical activity of the brain of a patient from a data base,
- preprocessing, by the computing system, the one or more electroencephalograms; and
- applying, by the computing system, a deep learning model to the preprocessed one or more electroencephalograms to generate tremor data indicating whether the one or more electroencephalograms represent one or more occurrences of one or more tremors of a patient.

Thus, the present invention allow to detect involuntary movement, such as tremors of a person. Such a tremor can be a rhythmic trembling of a part of the body.

In alternative aspects of the present invention, instead of generating tremor data, one of the following data can be generated: seizure data indicating whether the one or more electroencephalograms represent one or more occurrences of one or more seizures of a patient, migraine data indicating whether the one or more electroencephalograms represent one or more occurrences of one or more migraines of a patient, pain episode data indicating whether the one or more electroencephalograms represent one or more occurrences of one or more pain episodes of a patient.

Furthermore, in alternative aspects of the present invention, instead of acquiring EEGs, one or more spinal cord compound action potentials can be obtained, preprocessed and input to the deep learning model to generate said tremor data. Likewise, instead of acquiring EEGs, a photo plethysmography signal (PPG) can be obtained. PPG can detect oxygenation as well as correlates of blood pressure.

Particularly, a tremor of the hands is symptomatic of tremors. The invention solves the task of detecting acute tremors, or predicting the onset of a tremor by either data from at least one electroencephalogram (EEG) or data from at least one EEG and data from at least one wearable device with a deep learning algorithm (deep learning). The data can be measured on a patient-wide basis as well as on a patient-individual basis.

According to a preferred embodiment of the method according to the present invention, preprocessing the one or more electroencephalograms comprises: generating, by the computing system, one or more temporally-modified electroencephalograms by modifying a temporal resolution of the one or more electroencephalograms to match an expected temporal resolution of a deep learning model; and generating, by the computing system, one or more preprocessed electroencephalograms by subtracting a mean of samples of the one or more temporally-modified electroencephalograms from the samples of the one or more temporally-modified electroencephalograms.

Furthermore, according to a preferred embodiment of the method according to the present invention, preprocessing the one or more electroencephalograms comprises: generating, by the computing system, one or more temporally-modified electroencephalograms by modifying a temporal resolution of the one or more electroencephalograms to match an expected temporal resolution of the deep learning model; generating, by the computing system, one or more mean-subtracted electroencephalograms by subtracting a mean of samples of the one or more temporally-modified electroencephalograms from the samples of the one or more temporally-modified electroencephalograms; computing, by the computing system, a moving signal envelope over a sliding window of the one or more mean-subtracted electroencephalograms; and normalizing, by the computing system, the one or more mean-subtracted electroencephalograms based on the moving signal envelope.

According to yet another preferred embodiment of the method according to the present invention, the tremor data is first tremor data; wherein the method further comprises: obtaining, by the computing system, a marker channel; preprocessing, by the computing system, the marker channel; applying, by the computing system, a marker channel-based deep learning model to the preprocessed marker channel to generate second tremor data indicating a second set of occurrences of the one or more tremors; determining, by the computing system, whether the first tremor data and the second tremor data are consistent; and at least one of: adjusting, by the computing system, a confidence level of an occurrence of one of the tremors based on whether the occurrence of the tremor is in both the first set of occurrences and the second set of occurrences or only in one of the first set of occurrences and the second set of occurrences, or extending, by the computing system, based on the occurrence of the tremor being in both the first set of occurrences and the second set of occurrences, a duration of a monitoring session of a medical device that generates the one or more electroencephalograms.

According to a further preferred embodiment of the method according to the present invention, preprocessing the one or more electroencephalograms comprises: scaling, by the computing system, the waveform represented by the one or more electroencephalograms; and decomposing, by the computing system, the waveform represented by the one or more electroencephalograms into a plurality of channels corresponding to different frequency bands, and applying the deep learning model to the one or more preprocessed electroencephalograms comprises applying, by the computing system, the deep learning model to the channels to generate the arrhythmia data.

Furthermore, according to preferred embodiment of the method according to the present invention, preprocessing the one or more electroencephalograms comprises: scaling, by the computing system, the waveform represented by the one or more electroencephalograms; and generating, by the computing system, a transformed waveform by transforming the scaled waveform into a 2-dimensional time-frequency domain, and applying the deep learning model to the one or more preprocessed electroencephalograms comprises applying the deep learning model to the transformed waveform.

According to a further preferred embodiment of the method according to the present invention, preprocessing the one or more electroencephalograms comprises: determining, by the computing system, a polarity of the one or more electroencephalograms; and based on the polarity of the one or more electroencephalograms not being an expected polarity for the deep learning model, reversing, by the computing system, the polarity of the one or more electroencephalograms.

According to yet another preferred embodiment of the method according to the present invention, the deep learning model is a first deep learning model, and determining the polarity of the one or more electroencephalograms comprises applying, by the computing system, a second deep learning model to the one or more electroencephalograms to determine the polarity of the one or more electroencephalograms.

Furthermore, according to a preferred embodiment of the method, the latter further comprises: obtaining, by the computing system, training input vectors, wherein each of the training input vectors comprises a segment of a training electroencephalogram and device classification data that indicate one or more tremors detected in the training electroencephalograms; training, by the computing system, an autoencoder based on the training input vectors to reconstruct training electroencephalograms of the training input vectors; obtaining, by the computing system, additional device classification data; providing, by the computing system, the one or more preprocessed electroencephalograms and the additional device classification data to an input layer of the autoencoder; and determining, by the computing system, based on probability values generated by an intermediate layer of the autoencoder, whether the classification data correctly identifies tremors, if any, in the preprocessed electroencephalograms, wherein each of the probability values corresponds to a different tremor and indicates a level of confidence that the one or more preprocessed electroencephalograms contain the tremor.

According to yet another preferred embodiment of the method, preprocessing the one or more electroencephalograms comprises one or more of: scaling samples of a signal of the one or more electroencephalograms such that the samples of the signal of the one or more electroencephalograms are distributed in an expected range of sample values for the deep learning model, or increasing or decreasing a sample rate of the signal of the one or more electroencephalograms to match an expected sample rate of the deep learning model.

A further aspect of the present invention relates to a computing system comprising: a storage device configured to store one or more electroencephalograms that represent a waveform of a spontaneous electrical activity of the brain of a patient; one or more processing circuits configured to: preprocess the one or more electroencephalograms; and apply a deep learning model to the one or more preprocessed electroencephalograms to generate tremor data indicating whether the one or more electroencephalograms represent one or more occurrences of one or more tremors.

According to a preferred embodiment of this computing system, the one or more processing circuits are configured to perform the above-described method according to the present invention.

According to yet another aspect of the present invention, a (particularly non-transitory) computer-readable storage medium is disclosed having instructions stored thereon that, when executed on a computing system, cause the computing system to: obtain one or more electroencephalograms that represent a waveform of a spontaneous electrical activity of the brain of a patient; preprocess the one or more electroencephalograms; and apply a deep learning model to the one or more electroencephalograms to generate tremor data indicating whether the one or more electroencephalograms represent one or more occurrences of one or more tremors.

According to a preferred embodiment of this computer-readable storage medium, the instructions, when executed, cause the computing system to perform the above-described method according to the present invention.

Furthermore, according to yet another aspect of the present invention a further method is disclosed, the method comprising the steps of:
- obtaining, by a computing system, one or more electroencephalograms (EEG) that each represent a waveform of a spontaneous electrical activity of the brain of a patient (or of a population of patients), and/or obtaining, by at least one wearable device configured to be worn by a patient outside the body of the patient, wearable device data being indicative of a tremor of the patient wearing the wearable device,
- preprocessing, by the computing system, the one or more electroencephalograms and/or said wearable device data; and
- applying, by the computing system, a deep learning model to the preprocessed one or more electroencephalograms and/or to said wearable device data to generate tremor data indicating whether the one or more electroencephalograms and/or said wearable device data represent one or more occurrences of one or more tremors of a patient.

Thus, according to this further method, the data provided by the electroencephalograms (EEG) is combined with data coming from a wearable device worn by the respective patient outside the body of the patient. This data is denoted as wearable device data.

According to a preferred embodiment of this further method according to the present invention, the wearable device is one of: a smartwatch, a smart necklace, a smart anklet. Particularly, each of these smart devices is characterized in that it comprises a user interface for displaying information to a user (e.g. patient) and receiving input from the user, and a processor configured to execute a computer program that conducts obtaining the wearable device data, and particularly to preprocess and/or transmit said wearable device data or preprocessed wearable device data to a remote device, particularly to processing units of a computing system that can comprise a server (e.g. in a service center). Particularly, a server is a computer that provides a service to another computer which is denoted as client.

Furthermore, according to a preferred embodiment of the further method, the wearable device data represents a movement of a body part of the patient wearing the wearable device. Particularly said movement of the body part can be a tremble of one of: a hand of the patient wearing the wearable device, a head of the patient wearing the wearable device, a foot of the patient wearing the wearable device.

According to yet another preferred embodiment of the further method according to the present invention, obtaining one or more electroencephalograms, corresponds to obtaining multiple electroencephalograms of a spontaneous electrical activity of the brain of an individual patient or obtaining multiple electroencephalograms of a spontaneous electrical activity of the brains of a population of patients, and/or wherein obtaining by the at least one wearable device said wearable device data corresponds to obtaining by multiple wearable devices, each configured to be worn by a respective patient outside the body of the respective patient, wearable device data being indicative of a tremor of the respective patient wearing the respective wearable device.

Further, according to a preferred embodiment of the further method, obtaining one or more electroencephalograms is one of: conducted repeatedly over a certain time period, triggered by an event, triggered at a certain time, triggered at a certain location; and/or wherein obtaining the wearable device data is one of: conducted repeatedly over a certain time period, triggered by an event, triggered at a certain time, triggered at a certain location; and/or wherein obtaining the wearable device data.

According to yet another aspect of the present invention, a further computing system is disclosed, the further computing system comprising: a storage device (i.e. of at least one server, particularly a server being located in a service center) configured to store one or more electroencephalograms that each represent a waveform of a spontaneous electrical activity of the brain of a patient (or of a population of patients), and/or configured to store wearable device data from at least one wearable device (of a patient or of a population of patients), the wearable device data being indicative of a tremor of a patient wearing the respective wearable device; one or more processing circuits configured to: preprocess the one or more electroencephalograms and/or the wearable device data; and apply a deep learning model to the one or more preprocessed electroencephalograms and/or to the preprocessed wearable device data to generate tremor data indicating whether the one or more electroencephalograms and/or the wearable device data represent one or more occurrences of one or more tremors.

According to a preferred embodiment of the further computing system according to the present invention, the further computing system is configured to provide a medical device and/or the wearable device with the deep learning model and/or with data generated by the deep learning model, wherein the medical device is configured to acquire one or more electroencephalograms of a patient, and wherein the wearable device is configured to acquire wearable device data of a patient, the wearable device data being indicative of a tremor of the patient.

According to a preferred embodiment of the computing system according to one of the claims 20 to 22, the one or more processing circuits (e.g. of one or several servers, e.g. of a service center) are configured to perform the method according to one of the claims 15 to 19.

According to yet another aspect of the present invention, a (particularly non-transitory) computer-readable storage medium having instructions stored thereon is disclosed that, when executed, cause a computing system to: obtain one or more electroencephalograms that represent a waveform of a spontaneous electrical activity of the brain of a patient (or of a population of patients) and/or obtain, by at least one wearable device configured to be worn by a patient outside the body of the patient, wearable device data being indicative of a tremor of the patient wearing the wearable device; preprocess the one or more electroencephalograms and/or the wearable device data; and apply a deep learning model to the one or more electroencephalograms and/or the wearable device data to generate tremor data indicating whether the one or more electroencephalograms and/or the wearable device data represent one or more occurrences of one or more tremors.

According to a preferred embodiment of this computer-readable storage medium, the instructions, when executed, cause the computing system to perform the method according to one of the claims 15 to 19.

In the following, embodiments of the aspects of the present invention as well as further features and advantages of the present invention are described with reference to the Figures, wherein
- Fig. 1: an embodiment of a computing system and method according to the present invention using a deep learning module applied to EEGs to detect tremors of a person; and
- Fig. 2: shows a further embodiment of a computing system and method according to the present invention wherein at least one wearable device worn by a patient can further be used to provide wearable device data as input for the deep learning model.

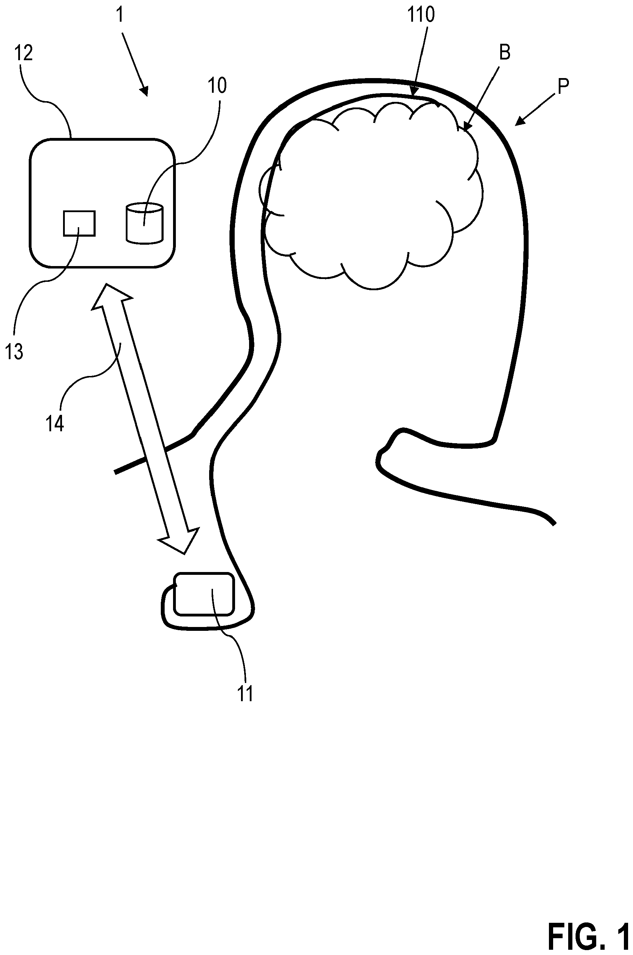

Fig. 1 shows a schematic illustration of an embodiment of the present invention, namely a computing system 1 comprising a storage device 10 configured to store one or more electroencephalograms (EEG) that represent a waveform of a spontaneous electrical activity of the brain B of a patient P. The storage device 10 can be part of a computer 12 such as remote server 12. In this regard, the notion computer or server also encompasses a plurality of computers operating jointly. Furthermore, the system 1 comprises one or more processing circuits 13 configured to preprocess the one or more electroencephalograms and to apply a deep learning model to the one or more preprocessed electroencephalograms to generate tremor data indicating whether the one or more electroencephalograms represent one or more occurrences of one or more tremors. Said processing circuits 13 can be comprises by said computer(s) 12, particularly said server(s) 12. Particularly, the computing system 1 can comprise a medical device 11, particularly an implantable medical device 11 implanted in the patient P, which medical device 11 is configured to acquire said one or more EEGs. Particularly, the medical device 11 is an implantable neurostimulator 11, particularly brain pacemaker, that is also configured to perform neurostimulation of the brain B of the patient. Particularly, the EEGs can be acquired from a single patient. In addition, the EEGs may also be acquired from a population of patients, and data deducted therefrom can be used on individual patients to detect tremors based on the deep learning model. In a further aspect of the present invention, which will be described further below, data from wearable devices worn by the patient (or a population of patients) can be used in addition or alternatively as an input to the deep learning model.

As shown in Fig. 1, the medical device 11 (e.g. neurostimulator) comprises electrodes 110 for acquiring the respective EEG and particularly for applying therapy in form of neurostimulation to the patient P. Furthermore, the medical device 11 is configured to communicate with computer (e.g. remote server) 12 via a wireless (e.g. radio-based) communication 14 that can involve at least one further device and/or at least one communication network. Particularly, the medical device 11 can comprise communication circuitry that may include one or more processors, memory, wireless radios, antennae, transmitters, receivers, modulation and demodulation circuitry, filters, amplifiers or the like for radio frequency communication with external devices such as computer / server 12. Furthermore, wireless communication 14 can be performed via an external device (not shown) such as a mobile device (e.g. external programmer, external patient device etc.). The medical device 11 can be configured to use said wireless communication to receive data from computer 12 and/or send data to computer 12.

Furthermore, particularly, the processing circuits 13 that may form part of computer / server 12 are configured to apply artificial intelligence (AI) techniques to analyze the acquired EEGs. Example AI techniques may include deep learning or other machine learning techniques. Neural network algorithms are one example of deep learning algorithms/models. The deep learning model / algorithm can be carried out on computer / server 12, but may also be carried out, either partially or completely, on other devices that can receive the necessary input data from the medical device 11. Such input data may be gathered separately and provided to the deep learning model which then processes actual EEGs received from a patient to detect tremors.

The computing system 1 is configured to trained to identify one or more aspects of the EEG of the patient P that are of interest in a given EEG with respect to detection of a tremor of the patient P. Said one or more aspects of the EEG include for instance signals with a predetermined frequency, amplitude or signal morphology. The deep learning model, or several such models, can be trained on EEGs drawn from a population of patients, individual patients, cohorts of patients, and other data.

Furthermore, the performance of the deep learning model may be improved by preprocessing the EEGs provided to the deep learning model as input. Particularly, preprocessing the data may enable the deep learning model to be used with EEG data generated by multiple types of devices. Thus, the computing system 1 may obtain an EEG that represents a waveform of a spontaneous electrical activity of the brain B of the patient P. Additionally, the computing system may preprocess the EEG. The computing system 1 using processing circuits 13 may then apply the deep learning model to the preprocessed EEG data to generate tremor data indicating whether the EEG represents one or more occurrences of one or more tremors.

In all embodiments of the various computing systems and methods described herein, the respective deep learning model can be implemented using one or more neural network systems, deep learning systems, or other types of supervised or unsupervised machine learning systems. For example, the deep learning model can be implemented by a feedforward neural network, such as a convolutional neural network, a radial basis function neural network, a recurrent neural network, a modular or associative neural network. Particularly, the computing system can train the deep learning model with patient data, including EEGs, for one or more populations of patients to generate data regarding one or more aspects of a tremor of the patients in the populations. Particularly, once the computing system 1 has pre-trained the deep learning model, the computing system 1 can further train the deep learning model with patient data specific to the patient P or a smaller cohort of patients.

Furthermore, the computing system 1 can train the deep learning model with the patient data for a population of patients, determines an error rate of the deep learning model 454, and then feeds the error rate back to deep learning model so as to allow' the deep learning model to update its predictions based on the error rate. In some examples, the error rate may correspond to differences between output data determined by deep learning model based on input data and prelabeled output data for the same input data. Particularly, the computing system 1 may use an error function to determine the error rate. The error function may be implemented using signal processing techniques in the manner conventionally used to detect occurrences of tremors. Particularly, the computing system 1 may receive from patient P or another person feedback indicating whether a detected tremor occurred in patient 14 within a particular time period. Furthermore, computer / server 12 may receive, from the medical device 11, a message indicating that the medical device 11 has detected (or has not detected) an occurrence of tremor in patient 14. Furthermore, computing system 1 may obtain the feedback in other ways, such as by periodically checking electronic medical records (EMR) data to determine if a tremor has occurred. The computing system 1 may update the deep learning model with the feedback. Thus, the training process may occur iteratively so as to incrementally improve the data generated by the deep learning model by "learning" from correct and incorrect data generated by the deep learning model in the past.

Further, the training process may be used to further fine-tune the deep learning model that is trained using population-based EEG data to generate more accurate data for a particular individual patient P.

Furthermore, the deep learning model cam also be implemented using a neural network. The neural network may include an input layer, and output layer, and one or more hidden layers between the input layer and the output layer. Each layer of the neural network may include one or more artificial neurons. The input layer of the neural network includes a plurality of input artificial neurons. The input layer may include a separate input artificial neuron for each sample value of a segment of an EEG. Particularly, the segment can represent the whole EEG waveform. Furthermore, the segment may be a subsegment of the EEG. For instance, in case the EEG comprises a waveform being 45 seconds in length, the segment may comprise a portion of the waveform representing the first 10 seconds of the EEG.

Furthermore, the computing system can provide overlapping segments of the EEG to the deep learning model. For example, the computing system 1 can provide a segment comprising samples representing seconds 0 through 10 of an EEG, then provide a segment comprising samples representing seconds 5 through 15 of the EEG, then provide a segment comprising samples representing seconds 10 through 20 of the EEG, and so on. In some examples, the computing system 1 may provide a segment that spans two or more EEGs. For ease of explanation, application of the deep learning model to an EEG may in fact refer to application of the deep learning model to a segment of the EEG.

Furthermore, particularly, the deep learning model comprises a convolutional neural network (CNN). For instance, in a preferred embodiment, a convolutional layer may follow an input layer of the type described above. A first convolutional layer artificial neuron may receive input from a first set of input layer artificial neurons consisting of a given number of consecutive input layer artificial neurons; a second convolutional layer artificial neuron may receive input from a second set of input layer artificial neurons consisting of the same given number of consecutive input layer artificial neurons, but offset from the first input layer artificial neuron of the first set of input layer artificial neurons by a stride length; a third convolutional layer artificial neuron may receive input from a third set of input layer artificial neurons consisting of the same given number of consecutive input layer artificial neurons, but offset from the first input layer artificial neuron of the second set of input layer artificial neurons by the stride length; and so on. The given number of consecutive input artificial neurons and the stride length are different hyperparameters of the CNN. One or more fully connected hidden layers may follow the convolutional layer.

Furthermore, for each respective tremor of a set of one or more tremors, the deep learning model implemented in computing system 1 may generate data that indicate whether one or more occurrences of the respective tremor are presented in a segment of an EEG. For instance, a hidden layer of the deep learning model can provide input data to the output layer of the deep learning model. For each respective tremor of the set of tremors, the output layer of the deep learning model can include a separate output artificial neuron corresponding to the respective tremor. The output artificial neuron corresponding to the respective tremor receives input data from a single artificial neuron in the hidden layer of deep learning model that also corresponds to the respective tremor. The data generated by the hidden layer artificial neuron corresponding to the respective tremor comprises a probability value indicating a probability that an occurrence of the tremor has happened in the segment of the EEG. An activation function of the output artificial neurons may apply a thresholding function to the probability values generated by the hidden layer artificial neurons. For each output artificial neuron, the thresholding function may cause the output artificial neuron to generate a first value (e.g., 1) if the probability value provided to the output artificial neuron is greater than a threshold and to generate a second value (e.g., 0) if the probability value provided to the output artificial neuron is less than the same threshold.

Furthermore, in the example of the previous paragraph, the computing system 1 may use the probability values generated by the hidden layer to track where occurrences of tremors happen within an EEG. For instance, as mentioned above, an EEG may be subdivided into segments and the computing system 1 can provide the segments to the deep learning model as input. Thus, by determining which segment of the EEG resulted in highest probability values corresponding to a tremor, the computing system 1 may determine which segment most likely represents the occurrence of the tremor.

As already described, the input provided to the deep learning model may include patient data in addition to EEGs. For instance, this patient data may additionally include wearable device data being indicative of movements of the patient P. Also, this data can in general be gathered for a population of patients or for an individual patient P.

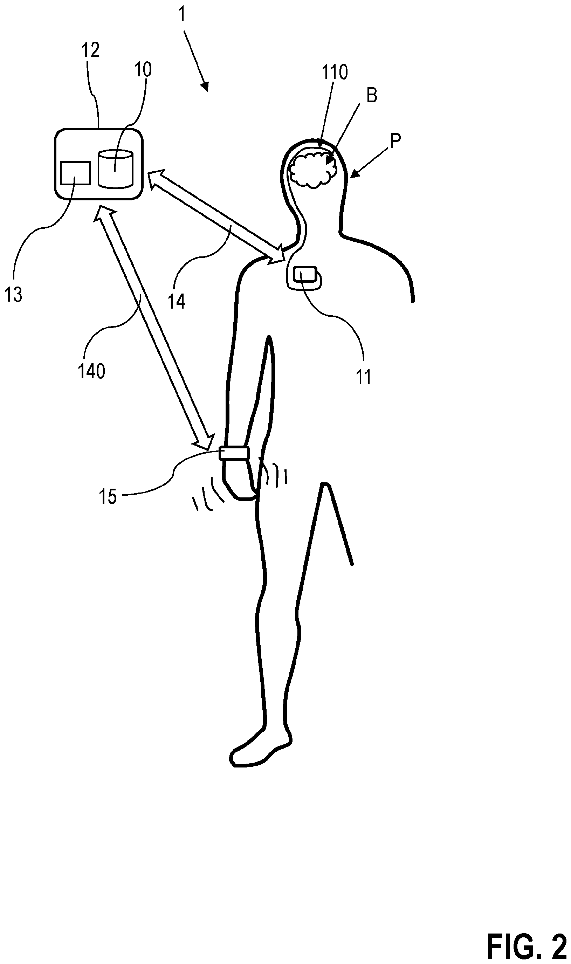

An embodiment of such a computing system 1 and method according to the present invention is shown in Fig. 2, wherein in addition to the components described in conjunction with Fig. 1, the computing system 1 also comprises a wearable device 15 such as a smart watch. Other wearable devices, particularly as described herein, may also be used.

Particularly, the wearable device 15 can be configured to measure the acceleration/deceleration of a hand of the patient P, so that tremors involving the hand and/or arm of the person can be detected by the wearable device 15. Wearable device 15 can be configured to transmit corresponding wearable device data being indicative of such movements to the computer / server 12 of the computing system.

The deep learning model or comparison date from a deep learning model can be stored in storage device 10 of the computing system and/or in a memory of the wearable device. Particularly, the wearable device 15 is configured to use the deep learning model and/or said comparison data to recognize the hand movement as a specific tremor, or as a symptom of a tremor. Particularly, in a preferred embodiment, as shown in Fig. 2, the patient(s) P wear the medical device 11 (e.g. implantable neurostimulator or brain pacemaker) and the wearable device 15. Here, wearable device data is transmitted from the wearable device (e.g. smart watch) to the remote computer / server 12 (e.g. of a service center) via wireless communication 114 that can be configured as wireless communication 14 described above. As described before, the acquired EEGs are transmitted from the medical device 11 to the computer / server 12 as well. Both data types (EEGs and wearable device data) are used as input to the deep learning model and to update the latter.

Furthermore, according to a preferred embodiment, the deep learning model or a part thereof can also be implemented on the wearable device, wherein the wearable device 15 is configured to evaluate at least a portion or individual tasks of the deep learning model. Alternatively, the wearable device transmits all the wearable device data to said remote computer / server 12 of the service center, where it is monitored, so that in case of a tremor detection by the deep learning model, a message is sent to the patient P informing him of the detected tremor(s).

Furthermore, with respect to Fig. 2, according to a further embodiment, the patient(s) P can only wear the wearable device 15, but not the medical device 11. Alternatively, according to yet another embodiment, the patient(s) can only have implanted the medical device 11, but does/do not wear the wearable device 15.

The present invention offers an advantageous computing system and method for detecting disorders in a patient's nervous system associated with a rhythmic trembling of a body part (tremor). A tremor of the hands is symptomatic of tremors. The invention allows to detect acute tremors, or predicting the onset of a tremor by analyzing either data from at least one electroencephalogram (EEG) or data from at least one EEG and a wearable device using a deep learning algorithm. The data can be measured on a patient-wide basis as well as on individual patients.
Particularly, the present invention offers the advantage, that a single deep learning model can be used for a variety of patients. Particularly, patients who do not yet have implanted the medical device 11 (e.g. brain pacemaker) can be monitored regarding tremors via their respective wearable device that can use a deep learning model that has also been trained with EEG data of other patients. Particularly, due to the monitoring of tremors based on wearable devices surgery can be avoided or postponed. Overall, the detection of tremors is more precise, time persistent and can be put in relation to specific events. Thus, care can be given to patients that live remotely or are difficult to reach. Furthermore, the employed wearable device is less expensive compared to a brain pacemaker / neurostimulator.

Particularly, according to a further aspect of the present invention, instead of an EEG one of the following quantities can also be used in the computing systems, methods, or computer-readable storage mediums according to the present invention: a spinal cord compound action potential, a photo plethysmography signal (PPG). PPG can detect oxygenation as well as correlates of blood pressure. Instead of tremors, the data can be evaluated in view of one of: seizures, migraine, or pain episodes

## Claims

1. A method, comprising the steps of:
- obtaining, by a computing system (1), one or more electroencephalograms (EEG) of a spontaneous electrical activity of the brain (B) of a patient (P) from a data base,
- preprocessing, by the computing system (1), the one or more electroencephalograms; and
- applying, by the computing system (1), a deep learning model to the preprocessed one or more electroencephalograms to generate tremor data indicating whether the one or more electroencephalograms represent one or more occurrences of one or more tremors.

2. The method according to claim 1, wherein preprocessing the one or more electroencephalograms comprises:
generating, by the computing system (1), one or more temporally-modified electroencephalograms by modifying a temporal resolution of the one or more electroencephalograms to match an expected temporal resolution of a deep learning model; and
generating, by the computing system (1), one or more preprocessed electroencephalograms by subtracting a mean of samples of the one or more temporally-modified electroencephalograms from the samples of the one or more temporally-modified electroencephalograms.

3. The method according to claim 1, wherein preprocessing the one or more electroencephalograms comprises:
generating, by the computing system (1), one or more temporally-modified electroencephalograms by modifying a temporal resolution of the one or more electroencephalograms to match an expected temporal resolution of the deep learning model;
generating, by the computing system, one or more mean-subtracted electroencephalograms by subtracting a mean of samples of the one or more temporally-modified electroencephalograms from the samples of the one or more temporally-modified electroencephalograms; computing, by the computing system (1), a moving signal envelope over a sliding window of the one or more mean-subtracted electroencephalograms; and
normalizing, by the computing system (1), the one or more mean-subtracted electroencephalograms based on the moving signal envelope.

4. The method according to one of the claims 1 to 3, wherein: the tremor data is first tremor data; the method further comprises:
obtaining, by the computing system (1), a marker channel;
preprocessing, by the computing system (1), the marker channel;
applying, by the computing system (1), a marker channel-based deep learning model to the preprocessed marker channel to generate second tremor data indicating a second set of occurrences of the one or more tremors;
determining, by the computing system (1), whether the first tremor data and the second tremor data are consistent; and at least one of:
adjusting, by the computing system (1), a confidence level of an occurrence of one of the tremors based on whether the occurrence of the tremor is in both the first set of occurrences and the second set of occurrences or only in one of the first set of occurrences and the second set of occurrences, or extending, by the computing system (1), based on the occurrence of the tremor being in both the first set of occurrences and the second set of occurrences, a duration of a monitoring session of a medical device (11) that generates the one or more electroencephalograms.

5. The method according to one of the preceding claims, wherein: preprocessing the one or more electroencephalograms comprises:
scaling, by the computing system (1), a waveform represented by the one or more electroencephalograms; and
decomposing, by the computing system (1), the waveform represented by the one or more electroencephalograms into a plurality of channels corresponding to different frequency bands, and
applying the deep learning model to the one or more preprocessed electroencephalograms comprises applying, by the computing system (1), the deep learning model to the channels to generate the arrhythmia data.

6. The method according to one of the preceding claims, wherein: preprocessing the one or more electroencephalograms comprises:
scaling, by the computing system (1), the waveform represented by the one or more electroencephalograms; and
generating, by the computing system (1), a transformed waveform by transforming the scaled waveform into a 2-dimensional time-frequency domain, and
applying the deep learning model to the one or more preprocessed electroencephalograms comprises applying the deep learning model to the transformed waveform.

7. The method according to one of the preceding claims, wherein preprocessing the one or more electroencephalograms comprises: determining, by the computing system (1), a polarity of the one or more electroencephalograms; and
based on the polarity of the one or more electroencephalograms not being an expected polarity for the deep learning model, reversing, by the computing system (1), the polarity of the one or more electroencephalograms.

8. The method according to claim 7, wherein:
the deep learning model is a first deep learning model, and determining the polarity of the one or more electroencephalograms comprises applying, by the computing system (1), a second deep learning model to the one or more electroencephalograms to determine the polarity of the one or more electroencephalograms.

9. The method according to one of the preceding claims, wherein the method further comprises: obtaining, by the computing system (1), training input vectors, wherein each of the training input vectors comprises a segment of a training electroencephalogram and device classification data that indicate one or more tremors detected in the training electroencephalograms; training, by the computing system (1), an autoencoder based on the training input vectors to reconstruct training electroencephalograms of the training input vectors; obtaining, by the computing system (1), additional device classification data; providing, by the computing system (1), the one or more preprocessed electroencephalograms and the additional device classification data to an input layer of the autoencoder; and determining, by the computing system (1), based on probability values generated by an intermediate layer of the autoencoder, whether the classification data correctly identifies tremors, if any, in the preprocessed electroencephalograms, wherein each of the probability values corresponds to a different tremor and indicates a level of confidence that the one or more preprocessed electroencephalograms contain the tremor.

10. The method according to one of the preceding claims, wherein preprocessing the one or more electroencephalograms comprises one or more of: scaling samples of a signal of the one or more electroencephalograms such that the samples of the signal of the one or more electroencephalograms are distributed in an expected range of sample values for the deep learning model, or increasing or decreasing a sample rate of the signal of the one or more electroencephalograms to match an expected sample rate of the deep learning model.

11. A computing system (1) comprising: a storage device (10) configured to store one or more electroencephalograms that represent a waveform of a spontaneous electrical activity of the brain (B) of a patient (P); one or more processing circuits 13 configured to: preprocess the one or more electroencephalograms; and apply a deep learning model to the one or more preprocessed electroencephalograms to generate tremor data indicating whether the one or more electroencephalograms represent one or more occurrences of one or more tremors.

12. The computing system according to claim 11, wherein the storage device (10) and the one or more processing circuits (13) are comprised by one or more remote servers (12), particularly of a medical service facility.

13. A computer-readable storage medium having instructions stored thereon that, when executed, cause a computing system (1) to: obtain one or more electroencephalograms that represent a waveform of a spontaneous electrical activity of the brain (B) of a patient (P); preprocess the one or more electroencephalograms; and apply a deep learning model to the one or more electroencephalograms to generate tremor data indicating whether the one or more electroencephalograms represent one or more occurrences of one or more tremors.

14. The computer-readable storage medium of claim 13, wherein the instructions, when executed, cause the computing system (1) to perform the method according to one of the claims 1 to 10.

15. A computing system comprising: a storage device (10) configured to store one or more electroencephalograms that represent a waveform of a spontaneous electrical activity of the brain of a patient (P) and/or to store wearable device data from at least one wearable device (15) of a patient (P), the wearable device data being indicative of a tremor of a patient (P) wearing the at least one wearable device (15); one or more processing circuits configured to: preprocess the one or more electroencephalograms and/or the wearable device data; and to apply a deep learning model to the one or more preprocessed electroencephalograms and/or to the preprocessed wearable device data to generate tremor data indicating whether the one or more electroencephalograms represent one or more occurrences of one or more tremors.
